# FASCICULE DE BREVET EUROPEEN

(11) **EP 0 639 956 B1**
(45) Date de publication et mention de la délivrance du brevet: **19.03.1997**
(21) Numéro de dépôt: 93910094.7
(22) Date de dépôt: 07.05.1993
(51) Int. Cl.: A61B 19/00

(54) **DISPOSITIF D'EXTENSION DE TISSU VIVANT**
VORRICHTUNG ZUM STRECKEN VON LEBENDEM GEWEBE
DEVICE FOR STRETCHING LIVE TISSUE

(30) Priorité: 07.05.1992 FR 9205893
(43) Date de publication de la demande: 01.03.1995
(73) Titulaire: MXM, Société Anonyme, F-06600 Antibes (FR); Frechet, Patrick, F-75016 Paris (FR)
(72) Inventeur: FRECHET, Patrick, F-75016 Paris (FR); FRAISSE, Georges, F-06700 Saint-Laurent-du-Var (FR); CHARVIN, Guy, F-06600 Antibes (FR)
(74) Mandataire: Somnier, Jean-Louis
(86) Numéro de dépôt international: FR9300441
(87) Numéro de publication internationale: WO9321849

(56) Documents cités:
- EP-A- 0 279 534
- EP-A- 0 432 743
- DE-A- 3 738 859
- US-A- 4 676 245
- US-A- 4 955 395

## Description

La présente invention a pour objet un dispositif d'extension de tissu vivant.

Le secteur technique de l'invention est le domaine de la réalisation de matériaux chirurgicaux implantables dans le corps humain.

Une des applications principales de l'invention est la réalisation de dispositifs d'extension implantables pour une durée déterminée, sous le cuir chevelu pour résorber la calvitie, mais d'autres applications sont envisageables avec le dispositif de l'invention, chaque fois qu'il y a utilité et/ou nécessité d'étirement des tissus, comme par exemple pour résorber des plaies, pour lesquelles il faut pouvoir recouvrir la surface atteinte par une nouvelle peau, en particulier quand la peau précédente a été détruite ou accidentée, lors de brûlure, traumatisme etc....

En matière de calvitie, dont le nom médical est l'alopécie androgénétique, il est connu que cette perte de cheveux définitive touche un homme sur trois âgé de 50 ans.

Elle fait partie du groupe des alopécies cicatricielles : elle est liée à l'atrophie progressive et prématurée du follicule pileux, qui est la racine du cheveu, et d'origine génétique et héréditaire.

Aucun traitement médical ne permet jusqu'à présente de prévenir ou de traiter l'alopécie androgénétique et aucune solution thérapeutique d'ordre médical n'est concevable avant 30 à 50 ans.

Actuellement la seule thérapeutique efficace est chirurgicale, à ce jour près de 2 millions d'américains y ont eu recours : il s'agit d'une chirurgie de redistribution d'une partie des follicules sains programmés génétiquement pour durer toute la vie. En pratique ce sont les cheveux de la couronne hippocratique.

Pour cela, il existe trois techniques essentielles, à savoir, les implants, les réductions de tonsure et les lambeaux, qui consistent toutes à déplacer le cuir chevelu sain pour le substituer en partie ou en totalité au cuir chauve.

Seule la deuxième technique de réduction de tonsure qui est la plus récente, et qui nous intéresse, concerne la présente invention : elle permet une amélioration nette des résultats dans les alopécies androgénétiques étendues, qui représentent à elles seules la majorité des cas à traiter.

Cette technique consiste à supprimer une zone de peau chauve et à rapprocher les deux bords de la plaie, une fois décollées les surfaces situées sous le cuir chevelu, de part et d'autre de ladite plaie. Les deux bords de celle-ci sont alors refermés sous tension au moyen de fils de suture chirurgicaux provoquant un étirement du cuir qui se traduit par une augmentation de la surface chevelue au dépens de la surface chauve.

Ainsi, une bande de 3 cm de large de cuir chauve peut être éliminée en moyenne à chaque intervention : quatre à six réductions de tonsure sont alors souvent nécessaires, car il y a une limite à l'élasticité de la peau et on ne peut pas raisonnablement enlever plus de 3 cm à la fois sans risque de déchirure; cependant malgré ce nombre élevé d'interventions, et même si on augmente ce nombre, on n'améliore en rien le résultat, qui permet d'éliminer seulement environ la moitié de la surface chauve initiale.

Cette non réduction totale du cuir chevelu est liée à deux phénomènes essentiels qui se superposent :
- il y a toujours une distension ou étalement secondaire de 20 à 50% des surfaces chauves proches de la zone de tension maximum, c'est-à-dire où la plaie est suturée, qui génère donc une zone centrale qui reste toujours chauve : ceci est bien décrit et précisé dans l'ouvrage le plus important en la matière consacré à la chirurgie du cuir chevelu, intitulé "Hair transplantation" deuxième édition de 1987 par Monsieur Rolf NORDSTRÖM et Walter UNGER, en particulier pages 504 à 516, précisant les réductions de surface en pourcentage des surfaces concernées par la réduction de tonsure.

Une communication du Docteur Richard SHIELL de MELBOURNE en AUSTRALIE en Février 1992 lors d'un congrès international sur la chirurgie du cuir chevelu à LOS ANGELES, donne des indications chiffrées des résultats de réductions du cuir chevelu et ainsi confirme les limites de ladite technique.
- La souffrance, voire même la disparition définitive des follicules pileux ischémiés par suite de la tension des couches superficielles du cuir chevelu, qui perturbe ainsi jusqu'à arrêter ou freiner la circulation du sang, provoquant alors l'ischémie desdites follicules.

Du fait de ces deux phénomènes, il est alors nécessaire de compléter les interventions de réduction de tonsure par des implants, ce qui augmente bien sûr le coût et également la durée de l'ensemble de l'opération.

Pour éliminer ces phénomènes, il est utilisé depuis près d'une dizaine d'années, surtout sur le continent américain des ballonnets placés sous le cuir chevelu et que l'on gonfle progressivement à des intervalles de temps réguliers : le cuir chevelu est ainsi mis sous pression interne, se distend et s'expanse; quand son expansion est jugée satisfaisante, on peut opérer, comme indiqué précédemment en enlevant une bande si ce n'est la totalité de la zone chauve, telle que les lèvres des zones restantes puissent être recousues bord à bord en bonne place.

Ainsi, on peut éliminer toutes les surfaces de zones chauves, et les remplacer par les zones chevelues distendues par les ballonnets; cependant, il est alors nécessaire de procéder à des remplissages réguliers de l'ordre de deux à trois fois par semaine desdits ballonnets, pour assurer l'expansion progressive sans déchirement : ceci nécessite un contrôle régulier et, de toutes façons, la déformation de la tête obtenue rend cette technique inutilisable, à grande échelle; les critères et considérations d'esthétique, et la difficulté d'immobiliser des personnes pendant de longues périodes, enlèvent tout intérêt à cette technique.

En effet, celle-ci prend plusieurs mois pour obtenir un résultat satisfaisant et oblige alors la personne concernée à s'isoler pendant une partie de cette période. De plus, il y a des risques de rupture des ballonnets et de mauvais contrôle des zones d'expansion, celle-ci sollicitant l'ensemble de la surface : ces inconvénients se traduisent par un risque de nouvelle intervention en cours de traitement.

Cette technique de ballonnets, pour obtenir une expansion tissulaire, est également courante dans d'autres traitements chirurgicaux, nécessitant la production de peau servant alors à couvrir une perte de substance chez un patient, en particulier pour récupérer des zones détruites ou accidentées après brûlure ou traumatisme.

Un tel dispositif est par exemple décrit dans la demande de brevet FR. 2.615.397 du 20 Mai 1987 déposé par MARSEILLE ASSISTANCE PUBLIQUE sur un "dispositif ambulatoire de gonflage en continu à pression constante de ballonnets d'expansion tissulaire" et également dans la demande de brevet FR. 2.608.916 du 26 Décembre 1986 déposé par MM. MAI et CRASSAS sur une "prothèse à expansion tissulaire"; dans ces deux demandes de brevet, et comme dans beaucoup d'autres de par le monde, il est décrit essentiellement un corps souple fermé, en un matériau biocompatible, apte à se dilater sous l'effet d'une pression de fluide, et destiné à être inséré sous le tissu et/ou la peau à réparer.

Quand il s'agit ainsi de réparation, le désagrément esthétique des ballonnets est secondaire par rapport au but recherché, bien que de toutes façons, cela limite la mobilité des personnes concernées et leur confort, suivant l'endroit où ces ballonnets sont placés.

Ainsi, le problème posé est de pouvoir augmenter d'une valeur déterminée, une surface donnée d'un tissu vivant par allongement, grâce à la faculté de reproduction de ces cellules, sans déformation inesthétique du volume de la partie du corps recouverte par ledit tissu, en limitant l'étalement secondaire des zones que l'on ne désire pas solliciter, et avec le minimum de nombre d'interventions et sur une durée la plus courte.

Une solution au problème posé est un dispositif d'extension du tissu vivant comprenant au moins un moyen élastique dont les dimensions extérieures, dans sa position active pouvant permettre l'extension dudit tissu sont telles qu'une partie de son périmètre puisse correspondre sensiblement et se superposer aux bords d'une partie au moins de la surface de tissu à traiter, et au moins deux moyens d'accrochage solidaires chacun d'une extrémité d'un moyen élastique du dispositif, opposés l'un de l'autre dans le sens de la direction d'élasticité active dudit moyen, et permettant sa fixation dans ledit tissu le long desdits bords.

Dans un mode de réalisation ledit moyen élastique peut être une pièce souple, plane et d'épaisseur constante ou d'épaisseur variable, en matériau élastomère de type silicone.

Dans un autre mode de réalisation, ledit moyen élastique peut être constitué d'une enveloppe en deux parties de forme cylindriques, creuses, ouvertes au moins à une extrémité et coulissantes l'une dans l'autre, à l'intérieur de laquelle est situé tout système à ressorts, soit comprimable et tendant à écarter lesdites parties jusqu'à une position d'écartement maximum limité par tout système de butée, soit extensible et tendant à rapprocher lesdites parties.

Le premier mode de réalisation permet ainsi essentiellement une traction sur les extrémités et donc une mise sous tension des zones de tissu situées à l'extérieur du dispositif, même éloignées les unes des autres, alors que dans le premier type du deuxième mode de réalisation, l'effet de traction concerne la zone du tissu vivant situé entre les extrémités : un dispositif ou l'autre sera choisi en fonction du type d'intervention et de l'objectif recherché. D'autres moyens élastiques peuvent être également développés pour l'application à la réduction de la calvitie telle que pris en référence principale dans la présente invention, mais également en fonction d'autres types d'applications, comme en particulier le raffermissement de la peau, ou la récupération des zones détruites ou accidentées.

Le résultat est de nouveaux dispositifs d'extension de tissu vivant, satisfaisant aux différents objectifs du problème posé et répondant aux divers inconvénients cités précédemment dans les systèmes existants, qui sont plutôt des dispositifs d'expansion en volume, alors que ceux décrits dans la présente invention sont qualifiés d'extension ou encore appelés extenseurs, car ils concernent sélectivement un étirement de surface.

En effet, avec les dispositifs d'extension suivant la présente invention, nous retrouvons les avantages de l'étirement du tissu vivant tel que le cuir chevelu, observé avec les ballonnets, mais ici, l'immense intérêt est lié au fait que l'étirement et la distorsion progressive du tissu ou cuir chevelu n'est pas obtenu par l'augmentation de volume, et donc de la surface dans toutes les directions, mais par l'augmentation de la surface seule dans des directions données. Il n'y a donc pas, d'une part, de modifications morphologiques en particulier de la tête, pour ce qui concerne le cuir chevelu et d'autre part, d'étirement dans des directions inutiles.

Par ailleurs, comme les extrémités actives du dispositif d'extension, suivant la présente invention, peuvent être placées exactement aux bords des zones que l'on veut soumettre a la traction pour en modifier la surface, les zones que l'on ne veut pas étirer ne sont pas soumises directement à ladite tension : ainsi dans le cadre de l'étirement du cuir chevelu, on n'a pas d'étalement secondaire des zones chauves, ce qui améliore l'efficacité du système et sa rapidité d'action; et on peut tirer sur des zones chevelues éloignées du bord de la zone chauve, pour qu'en fin d'extension et donc lors du retour de l'extenseur à sa position repos, ses extrémités étant alors à une certaine distance l'une de l'autre, cette distance correspond à la longueur des zones chevelues situées et maintenues à l'intérieur des extrémités : une seule opération suffit donc effectivement pour atteindre le résultat recherché; par ailleurs, il n'y a pas de destruction folliculaire par suite d'ischémie des couches superficielles du cuir chevelu, comme cela peut être observé dans d' autres techniques.

Par rapport à la technique de base de réduction de tonsure, mais n'utilisant pas de ballonnets pour des raisons esthétiques, le nombre d'intervention est réduite à deux, comme théoriquement c'est le cas avec les ballonnets, mais avec des risques d'interventions intermédiaires, soit l'une pour la mise en place du dispositif, et l'autre pour son enlèvement, au lieu des quatre à six citées précédemment.

Par rapport à la technique de base de réduction de tonsure sans ballonnets, on peut également souligner la réduction de la durée du traitement qui est au maximum de un à trois mois contre douze mois ou plus : ceci est dû au fait que durant le temps d'une seule opération, il est impossible de vouloir tirer exagérément sur des tissus qui offrent une résistance initiale de plus de 15 kg/cm², alors que lors de tension permanente et progressive, les tissus s'adaptent, se régénèrent et s'allongent ainsi d'une façon relativement rapide.

Lié aux réductions de durée de traitement et du nombre d'interventions, le coût global du traitement est ainsi beaucoup diminué, de même que la douleur subie par la personne concernée, celle-ci étant d'autant plus satisfaite que la surface chauve éliminée est beaucoup plus importante que dans le cas d'opérations successives du fait de l'absence de l'étirement secondaire indiqué précédemment.

Le dispositif d'extension suivant l'invention est par ailleurs d'une extrême simplicité, tant dans sa conception que dans sa manipulation chirurgicale, ce qui en fait un dispositif fort intéressant, pour un très faible encombrement, de l'ordre de 1 mm d'épaisseur dans le cas de tissu souple, d'épaisseur constante, mais il peut être d'épaisseur variable et sa "plastique" parfaite et douce, s'adapte à chaque tête.

Il peut donc être utilisable dans tous les cas de calvitie étendue et également dans toutes les autres applications citées précédemment.

On pourrait citer d'autres avantages de la présente invention, mais ceux cités ci-dessus en montrent déjà suffisamment pour en démontrer la nouveauté et l'intérêt.

La description et les figures ci-après représentent des exemples de réalisation de l'invention, mais n'ont aucun caractère limitatif : d'autres réalisations sont possibles dans le cadre de la portée et de l'étendue de la présente invention, en particulier en changeant la forme et le type des moyens élastiques, ainsi que ceux des moyens d' accrochage.

La figure 1A est une vue schématique en perspective du dispositif suivant l'invention implanté sur un crâne.

La figure 1B est une vue en coupe partielle de l'accrochage du dispositif sous le cuir chevelu.

La figure 2A est une vue en plan d'un exemple du dispositif.

La figure 2B est une vue de profil du dispositif de la figure 2A.

La figure 2C est une vue en perspective d'un dispositif en deux parties avec accrochage central.

La figure 2D est une vue en perspective d'un dispositif avec un support de suture.

La figure 3A est une vue de dessus d'un autre dispositif réalisé par combinaison de moyens élastiques.

La figure 3B est une vue de profil du dispositif de la figure 3A.

La figure 3C est une vue de profil d'un dispositif suivant les vues 3A et 3B, mais représenté en épaisseur variable.

Les figure 4A et 4B sont des vues perspectives de deux exemples du dispositif de mise en place du dispositif suivant l'invention.

Les figures 5A et 5B sont deux vues perspectives de deux autres exemples de réalisation du dispositif de l'invention en matériau souple.

La figure 6A est une vue perspective d'un autre exemple de dispositif suivant l'invention.

La figure 6B est un dispositif réalisé par articulation de dispositifs de la figure 6A.

La figure 7 représente plusieurs autres exemples de réalisation du système d'accrochage.

La figure 1A est une vue perspective schématique de la tête d'une personne, dont le cuir chevelu comporte une zone chauve 1, et des zones chevelues 3 situées à la périphérie de cette zone chauve 1.

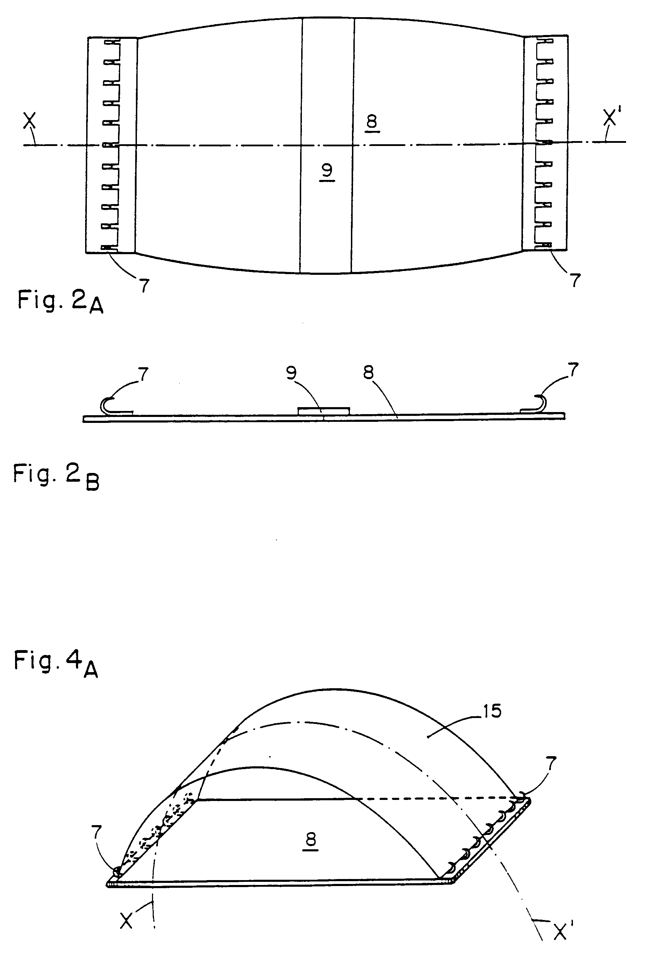

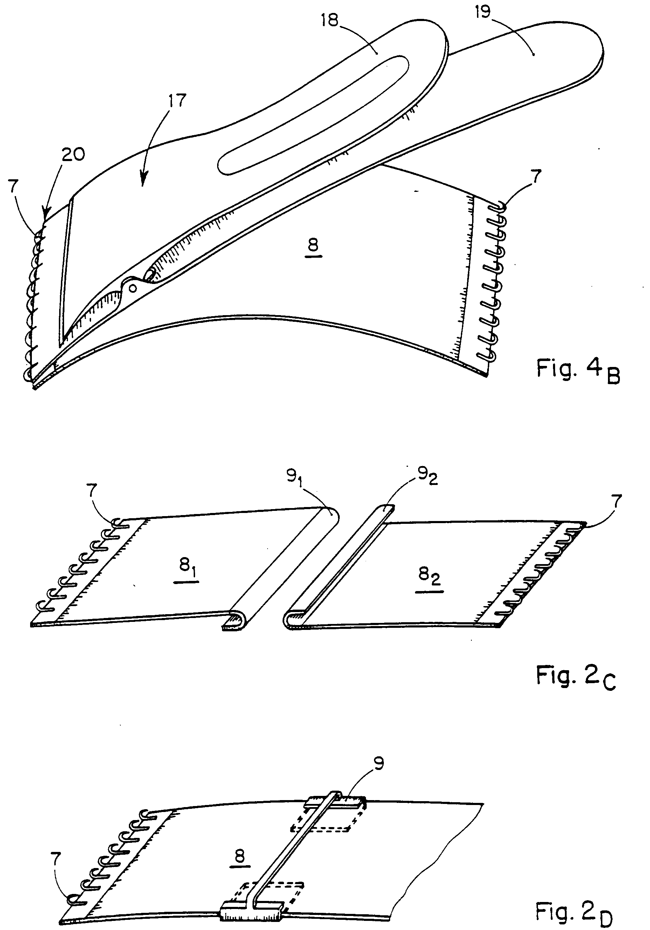

On place alors le dispositif d'extension 2 suivant la présente invention sous ledit cuir chevelu, par l'incision d'une ouverture 14 au milieu de ladite zone chauve 1 : les systèmes d'accrochage situés à deux extrémités opposées du dispositif 2 représenté ici comme celui de la figure 2A sous la forme d'un rectangle, et constitués par exemple de crochets 7 sont ancrés dans la galéa 6 et l'hypoderme, qui est la couche fibreuse rigide constituant la partie profonde du cuir chevelu, dont une coupe est donnée en figure 1B.

Lesdits systèmes d'accrochage 7 sont ainsi mis en place le long des bords 13 d'une partie au moins de la surface de tissu à traiter, dont l'exemple ici est celui du cuir chevelu, dont on veut rapprocher les zones 3 chevelues; ces bords 13 ne sont pas forcément ceux des bords limites 16 des surfaces séparant les zones chevelues 3 de la zone chauve 1; en effet, il est préférable, comme indiqué précédemment, de tirer sur des zones chevelues éloignées du bord de la zone chauve, pour qu'en fin d'extension, et lors du retour de l'extenseur 2 à sa position repos, ces extrémités étant alors à une certaine distance l'une de l'autre, cette distance correspondant à la longueur des zones chevelues 17 situées à l'intérieur de ces extrémités : cette répartition de traction sur les zones chevelues que l'on veut traiter, peut être effectuée d'une façon plus complète et plus efficace, suivant le dispositif tel que représenté dans les figures 3.

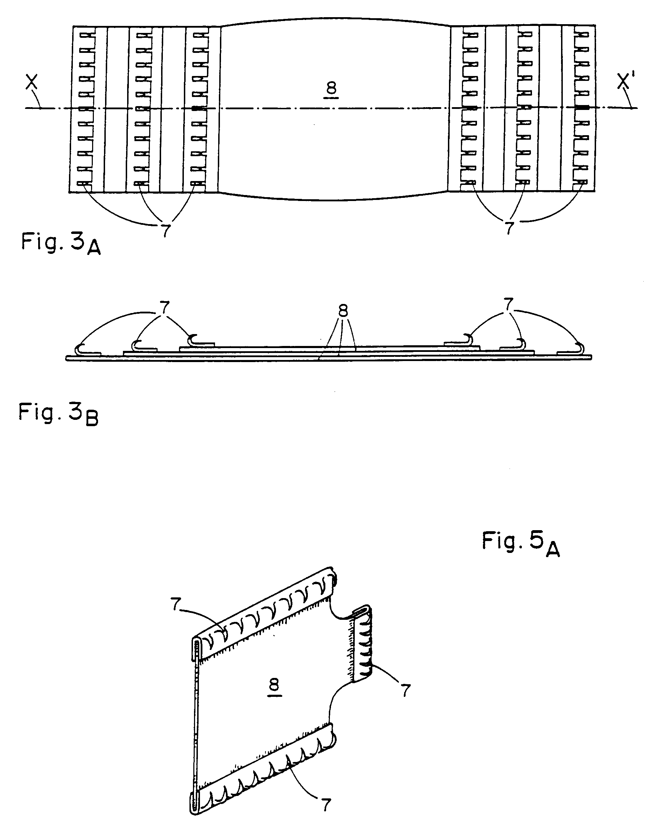

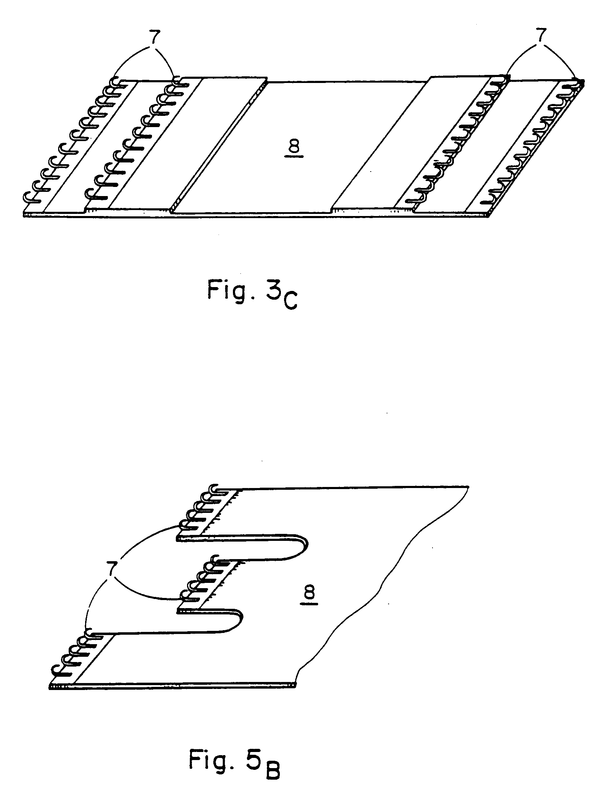

Il n'est pas non plus forcément utile et nécessaire de solliciter du reste d'une façon uniforme et continue toute la longueur des bords 13 de la surface à traiter, on ne peut tirer et s'accrocher que sur une partie et en des zones non adjacentes, suivant l'état du tissu et le résultat que l'on veut obtenir, grâce par exemple à un dispositif d'extension tel que représenté sur la figure 5B; les zones adjacentes comprises entre deux points ou deux lignes de traction seront en effet entraînées indirectement et plus ou moins suivant la disposition des systèmes d'accrochage 7, et ainsi, même en ne tirant que sur une partie des bords 13 de la zone à traiter, celle-ci sera concernées dans son ensemble par l'extension, mais d'une manière sélective, déterminée et définie, permettant d'obtenir le meilleur résultat souhaité.

Dans cet exemple de la figure 1A, le dispositif d'extension est un dispositif élastique, ou encore dit extenseur, réalisé en pièces souples planes en matériau élastomère de type silicone, qui est mis en tension lors de sa mise en place, à l'aide d'outils tels que ceux indiqués dans les figures 4 : il va alors progressivement se contracter dans le sens de sa direction d'élasticité active XX' ainsi sollicitée pour retrouver sa longueur initiale, en rapprochant ses extrémités et donc ses systèmes d'accrochage 7, et ainsi tendre à rapprocher lesdits bords 13 en comprimant la zone chauve 1 et suivant le cas une partie 17 des zones chevelues, comme indiqué précédemment, et située entre eux; des expériences confidentielles ont démontré que cette extension compression se réalise dans une durée de l'ordre de un à deux mois.

On peut alors réouvrir la cicatrice 14, qui a permis la mise en place dudit dispositif, pour enlever celui-ci et découper la bande chauve 1, qui est alors bien sûr en excès et recoudre les bords 16 directement entre eux.

On a obtenu ainsi l'effet recherché et aucune autre intervention n'est alors nécessaire. Pendant toute la durée de l'étirement et de l'activité du dispositif, celui-ci est alors invisible extérieurement, et seul un léger plissement de peau peut être perceptible, ce qui ne modifie pas d'une façon importante l'esthétique de la personne, qui peut ainsi continuer ses activités, sans être obligée de s'isoler du reste de son environnement, comme dans le cas des ballonnets.

La figure 1B est une vue en coupe du cuir chevelu dans lequel est ancré le dispositif suivant l'invention par un de ses systèmes d'accrochage 7, dans la galéa et hypoderme 6, qui est la surface fibreuse située sous le cuir chevelu 5 proprement dit, lui-même protégé par la peau extérieure 4 : dans l'exemple de la figure 1A, les systèmes d'accrochage 7 tendent à tirer les bords 13 de la zone extérieure à traiter 3 en la sollicitant ainsi en traction, alors qu'au contraire, la zone située à l'intérieur desdits bords 13 est en compréhension.

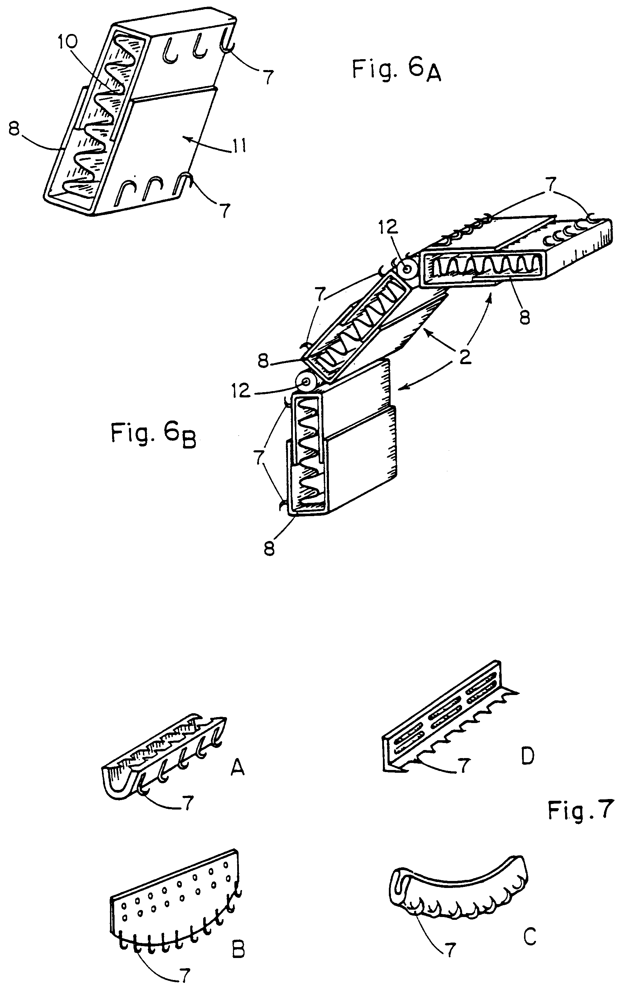

Dans l'exemple du dispositif suivant l'invention représenté dans les figures 6A et 6B, les systèmes d'accrochage sont situés en sens inverse des précédents, de façon à permettre au contraire un accrochage permettant la tension de la zone située entre eux et une compression des zones situées à l'extérieur, grâce à un système à ressort 10 comprimable et tendant à écarter ces systèmes d'accrochage.

La figure 2A est une vue de dessus d'un dispositif tel qu'implanté suivant la figure 1A, dans lequel ledit moyen élastique 8 est une pièce souple en matériau élastomère de type silicone ; la figure 2B est une vue de profil d'un tel dispositif avec, ladite pièce souple, plane, et d'épaisseur et de largeur constantes, mais elle pourrait être également d'épaisseur ou/et de largeur variable tel que représenté par exemple sur les figures 3C et 3B; sur cette figure, ladite pièce est réalisée au moins en deux parties séparables, correspondant et portant chacune à une extrémité du dispositif un desdits moyens d'accrochage 7 et lesdites parties séparables sont réunies entre elles par tout moyen de liaison 9. Ce dit moyen de liaison peut avoir trois fonctions :
- d'une part, permettre la mise en place du dispositif en au moins deux parties indépendantes l'une de l'autre par accrochage des systèmes d'extrémité dans le tissu à traiter, puis, la mise en tension des deux parties et leur liaison par ledit dispositif 9, sans nécessiter de moyens externes tel que celui représenté à la figure 4,
- d'autre part, de pouvoir recevoir un système adhésif ou renfort de suture tel que représenté sur les figures 2B et 2D permettant de maintenir "collée" ou suturée sur sa surface la zone chauve 1 qui est comprimée par ledit dispositif, sans risquer que celle-ci fasse des plis inesthétiques importants en se soulevant sous l'effet de la compression,
- et enfin de pouvoir être constituée d'un matériau plus résistant que la pièce élastique 8, afin de permettre lors de réintervention intermédiaire éventuelle, de couper le cuir chevelu, sans risque de couper ladite pièce élastique, comme représenté sur la figure 2D. Sur cette figure, on remarque que le renfort 9 de suture peut être utilisé même avec un extenseur 8 en une seule partie.

Ladite pièce de liaison 9 ou renfort de suture peut être en tissu de type polyester ou en plaques métalliques polyéthylène ou tout autre matériau biocompatible et résistant : elle peut venir soit recouvrir les deux extrémités des deux parties au milieu de l'extenseur 8, tel que représenté sur les figures 2A, 2B ou 2D, mais également, cette pièce de liaison 9 peut être constituée de deux éléments, chacun reliés à une des extrémités des deux parties de l'extenseur, tel que représenté sur la figure 2C sous forme d'un système à accrochage central.

Les figures 3A et 3B représentent une vue de dessus et de profil d'un autre exemple de dispositif d'extension de tissu vivant suivant la présente invention, comprenant plusieurs moyens élastiques 8 du type du précédent, en pièces souples, superposables, planes et indépendantes, d'épaisseur constante ou variable, de dimensions différentes, dont la plus grande est placée la plus éloignée de la surface dudit tissu à traiter, la suivante tant en taille qu'en position est placée contre la précédente, entre celle-ci et le tissu à traiter, et ainsi de suite jusqu'à la plus petite, qui est ainsi directement en contact avec ledit tissu sur toute sa surface.

Un tel dispositif permet ainsi de solliciter selon différentes lignes de traction plusieurs zones du tissu à traiter, et ainsi d'avoir une meilleure répartition des tensions et/ou suivant des forces différentes d'avoir des sollicitations d'extension du tissu différentes suivant les zones : ainsi dans le cas de la calvitie, les zones chevelues 3 peuvent être de densités de cheveux différents, et on peut ainsi homogénéiser celles-ci.

Dans le même objectif, les pièces souples planes constituant lesdits dispositifs peuvent être réalisées en épaisseur et largueur variables, de façon à solliciter certaines zones du tissu vivant ou d'autres plus ou moins : ceci permet d'avoir soit une seule pièce souple élastique sur laquelle peuvent être fixés les systèmes d'accrochage 7 en différents endroits, tel que représenté sur la figure 3C, sans nécessiter de couches superposées de pièces comme indiqué dans les figures 3A et 3B, soit avec de telles couches superposées, comme dans ces figures 3A et 3B, d'obtenir des effets d'étirement sélectifs complexes. Ceci complète et se combine avec la possibilité de disposer des systèmes d'accrochage en des points choisis, en ligne continue, tel que représenté sur les figures 2 et 3, ou discontinue, tel que représenté sur les figures 5, tout le long des bords 13 de la zone à traiter ou sur une partie seulement, comme indiqué précédemment et ci-après.

Les figures 4A et 4B représentent des vues perspectives de deux exemples d'outils de mise en place du dispositif d'extension, constitués par un moyen élastique 8.

Sur la figure 4A, celui-ci est représenté en position repos et devant être étendu et conformé à la forme par exemple d'un crâne grâce à un outil de mise en place 15 : on appuie celui-ci sur la partie du corps dont on veut que la pièce élastique 8 épouse la forme par déformation élastique sous traction de celle-ci, jusqu'à ce que lesdits systèmes d'accrochage 7 correspondent aux bords 13 de la surface du tissu à traiter et y soient crochetés permettant le retrait de ladite pièce 15. Celle-ci n'est cependant pas nécessaire quand ladite pièce souple est réalisée en deux parties séparables l'une de l'autre comme indiqué dans les figures 2A et 2B.

La figure 4B représente un autre outil 17 permettant la mise en place des systèmes d'accrochage 7 l'un après l'autre : il est constitué d'un bec 20 de prise desdits crochets ou systèmes d'accrochage 7, comme dans le cas d'un outil de la figure 4A, à l'extrémité d'un manche 19, et il comporte un poignet articulé 18 permettant de par sa forme, de pouvoir décoller le cuir chevelu ou le tissu vivant sous lequel on veut glisser ledit extenseur 8 en fermant ce dit poignet contre ledit manche de l'outil 19; ainsi cet outil permet non seulement la mise en place de l'extenseur, mais également sa possibilité de le retirer d'une façon assez simple et pratique.

Les figures 5 sont d'autres exemples de dispositif suivant l'invention, dans lequel ledit moyen élastique 8comporte trois moyens d'accrochage 7 : dans la figure 5A, deux des moyens d'accrochage 7 sont situés dans des directions opposées l'une de l'autre, comme dans les figures précédentes, et le troisième moyen est situé suivant une direction perpendiculaire, permettant par exemple de ramener également une zone chevelue de l'arrière du crane si nécessaire; dans le cas de la figure 5B, les trois moyens d'accrochage 7 indiqués constituent en fait un seul moyen d'accrochage 7 suivant la définition précédente, situé d'un seul côté d'une seule extrémité du moyen élastique 8, mais suivant des lignes non continues les unes par rapport aux autres, de façon à venir solliciter certaines zones plus que d'autres, sachant que les zones intermédiaires seront indirectement sollicitées comme expliqué précédemment.

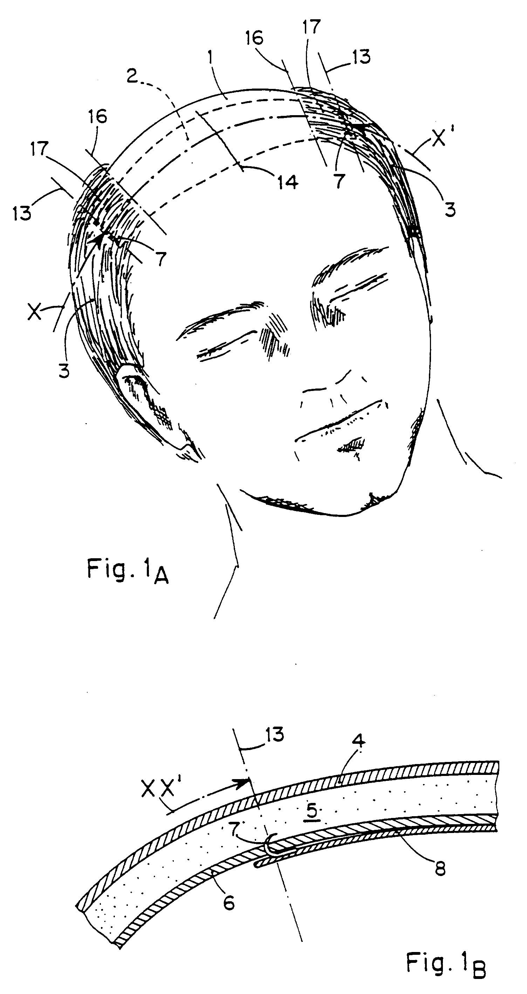

On peut également réaliser d'autres dispositifs avec quatre ou cinq, ou même plus dispositifs d'accrochage suivant l'objectif recherché, lesdits dispositifs 7 pouvant être suivant des lignes droites, tel que représenté dans les figures 1 à 6 et 7A et 7B, mais aussi courbes comme dans les figures 7B et 7C, ou sous toute autre forme en ligne continue ou discontinue comme indiqué ci-dessus, suivant les zones que l'on veut soumettre à la traction par rapport à l'ensemble de la zone à traiter, en fonction du résultat recherché et de la situation initiale..

Les figures 6A et 6B représentent un autre exemple de réalisation de dispositif suivant l'invention dans lequel ledit moyen élastique 8 est constitué d'une enveloppe 11 en deux parties semi fermées et coulissantes l'une dans l'autre, a l'intérieur de laquelle est situé à tout système à ressort 10 soit comprimable et tendant à écarter lesdits parties jusqu'à une position d'écartement maximum limité par tout système de butée, soit extensible et tendant à rapprocher lesdites parties. Dans le premier type, les systèmes d'accrochage 7 sont alors dirigés vers l'extérieur du dispositif, comme représenté sur la figure 6A, et dans le deuxième type, ils sont dirigés comme dans les exemples des figures 1 à 5. Sur la figure 6B, le dispositif suivant l'invention comprend plusieurs enveloppes 11 articulées les unes aux autres par des axes 12 permettant à l'ensemble de ces enveloppes 11 d'épouser la forme naturelle du tissu vivant à traiter.

La figure 7 représente plusieurs exemples de système d'accrochage 7, qui peuvent être constitués de crochets, de griffes, de pinces, ou tout autre système de liaison pouvant s' ancrer dans un tissu vivant et assurer la transmission de la tension du moyen élastique 8 et dont le système de liaison avec ces dits moyens élastiques est adapté à la configuration de celui-ci, soit par pinçage, soit par soudure, soit par couture, soit par clipsage....

Les dispositifs suivant l'invention peuvent être également implantés par voie externe, à condition cependant, dans le cas de la réduction de la calvitie indiquée en application ci-dessus, que les crochets soient placés dans la galéa quand même, à travers l'ensemble de l'épaisseur du cuir chevelu, de façon à faire revenir celle-ci et obtenir le résultat recherché, bien que l'effet esthétique obtenu sera cependant limité par la vue extérieure du dispositif suivant l'invention, mais qui peut être éventuellement camouflé par une perruque portable pendant la durée de cette traction compression, puisqu'il n'y a pas de changement de volume proprement dit et cela supprime une intervention pour la mise en place du dispositif.

Dans d'autres utilisations, les dispositifs suivant l'invention peuvent être retenus, chaque fois qu'il s'agit de vouloir tendre ou étendre la surface d'un tissu qui soit suffisamment résistant, pour éviter les déchirures et des ruptures du faisceau, et chaque fois que ledit étirement des tissu est souhaitable.

## Revendications

1. Dispositif d'extension de tissu vivant, caractérisé en ce qu'il comprend au moins un moyen élastique (8) dont les dimensions extérieures, dans sa position active pouvant permettre l'extension dudit tissu, sont telles qu'une partie de son périmètre puisse correspondre sensiblement et se superposer aux bords (13) de la surface de tissu à traiter, et au moins deux moyens d'accrochage (7) solidaires chacun d'une extrémité d'un moyen élastique (8) du dispositif, opposés l'un de l'autre dans le sens de la direction d'élasticité active dudit moyen (8), et permettant sa fixation dans ledit tissu le long desdits bords (13).

2. Dispositif d'extension de tissu vivant suivant la revendication 1, caractérisé en ce que ledit moyen élastique (8) est une pièce souple en matériau élastomère de type silicone.

3. Dispositif d'extension de tissu vivant suivant la revendication 2, caractérisé en ce que ladite pièce souple est réalisée au moins en deux parties séparables, correspondant et portant chacune à une extrémité dudit dispositif, un desdits moyens d'accrochage (7), et lesdites parties séparables sont réunissables entre elles par tout moyen de liaison (9).

4. Dispositif d'extension de tissu vivant suivant l'une quelconque des revendications 2 et 3, caractérisé en ce qu'il comprend plusieurs moyens élastiques (8) en pièces souples superposables, de dimensions différentes, dont la plus grande est placée la plus éloignée de la surface dudit tissu à traiter, la suivante en taille et en position est placée contre la précédente entre celle-ci et le tissu à traiter et ainsi de suite jusqu'à la plus petite.

5. Dispositif d'extension de tissu vivant suivant l'une quelconque des revendications 2 à 4, caractérisé en ce que chacune desdites pièces souples est d'épaisseur et de largeur variables, suivant les zones de sa surface.

6. Dispositif d'extension de tissu vivant suivant la revendication 1, caractérisé en ce que ledit moyen élastique (8) est constitué d'une enveloppe (11) en deux parties de forme cylindrique creuses, ouvertes au moins à une extrémité et coulissantes l'une dans l'autre, à l'intérieur de laquelle est situé tout système à ressort (10) comprimable et tendant à écarter lesdites parties jusqu'à une position d'écartement maximum limité par tout système de butée.

7. Dispositif d'extension de tissu vivant suivant la revendication 1, caractérisé en ce que ledit moyen élastique (8) est constitué d'une enveloppe (11) en deux parties semi fermées et coulissantes l'une de l'autre, à l'intérieur de laquelle est situé tout système à ressort (10) extensible et tendant à rapprocher lesdites parties.

8. Dispositif d'extension de tissu vivant suivant l'une quelconque des revendications 6 et 7, caractérisé en ce qu'il est constitué de plusieurs enveloppes (11) articulées les unes aux autres par des axes (12) permettant à l'ensemble de ces enveloppes (11) d'épouser la forme naturelle du tissu vivant à traiter.

## Claims

1. Device for extending living tissue, characterized in that it comprises at least one resilient means (8) whose outside dimensions, in its active position enabling said tissue to be extended, are such that a portion of its perimeter corresponds substantially to and is superposed with the edges (13) of the area of tissue to be treated, and at least two fastening means (7) each secured to an end of a resilient means (8) of the device, opposite to each other in the direction of active resilience of said means (8) and enabling it to be fixed to said tissue along said edges (13).

2. Extension device for living tissue according to claim 1, characterized in that said resilient means (8) is a flexible piece of silicone type elastomer material.

3. Extension device for living tissue according to claim 2, characterized in that said flexible piece is made of at least two separable portions, each corresponding to and carrying one of said fastening means (7) at one end of said device, said separable portions being unitable by any link means (9).

4. Extension device for living tissue according to any one of claims 2 and 3, characterized in that it comprises a plurality of resilient means (8) made up of superposable flexible pieces of different dimensions, of which the largest is placed furthest from the surface of said tissue to be treated, the next one in size and position being placed against the preceding piece between it and the tissue to be treated, and so on until the smallest piece.

5. Extension device for living tissue according to any one of claims 2 to 4, characterized in that each of said flexible pieces is of varying width and thickness, depending on the regions of its surface.

6. Extension device for living tissue according to claim 1, characterized in that said resilient means (8) is constituted by an envelope (11) made up of two hollow cylindrical portions that are open at at least one end and that slide one in the other, any appropriate compressible spring system (10) being located therein and tending to move said portions apart to a position of maximum separation limited by any abutment system.

7. Extension device for living tissue according to claim 1, characterized in that said resilient means (8) is constituted by an envelope (11) in two semi-closed portions sliding one in the other, any appropriate extensible spring system (10) being located therein and tending to move said portions towards each other.

8. Extension device for living tissue according to any one of claims 6 and 7, characterized in that it is made up of a plurality of envelopes (11) that are hinged to one another about axes (12) enabling the set of theses envelopes (11) to follow the natural shape of the living tissue to be treated.

## Patentansprüche

1. Vorrichtung zum Strecken von lebendem Gewebe, dadurch gekennzeichnet, daß sie mindestens eine elastische Vorrichtung (8) umfaßt, deren Außenabmessungen in ihrer aktiven Position, die das Strecken des Gewebes ermöglichen kann, derart sind, daß ein Teil ihres Umfangs im wesentlichen den Rändern (13) der Oberfläche des zu behandelnden Gewebes entsprechen und sich darüberlegen kann und mindestens zwei Befestigungsvorrichtungen (7), die jeweils fest verbunden sind mit einem Ende einer elastischen Vorrichtung (8) der Vorrichtung und einander in der Richtung gegenüberliegen, die die Richtung der aktiven Elastizität darstellt, und ihre Befestigung in dem Gewebe entlang der Ränder (13) ermöglichen.

2. Vorrichtung zum Strecken von lebendem Gewebe nach Anspruch 1, dadurch gekennzeichnet, daß die elastische Vorrichtung (8) ein dehnbares Element aus einem silikonartigen Elastomer ist.

3. Vorrichtung zum Strecken von lebendem Gewebe nach Anspruch 2, dadurch gekennzeichnet, daß das dehnbare Element mindestens aus zwei trennbaren Teilen gefertigt ist, die einander entsprechen und jeweils an einem Ende der Vorrichtung Befestigungsvorrichtungen (7) tragen, und daß die trennbaren Teile durch eine beliebige Verbindungsvorrichtung (9) miteinander verbunden werden können.

4. Vorrichtung zum Strecken von lebendem Gewebe nach einem der Ansprüche 2 und 3, dadurch gekennzeichnet, daß sie mehrere elastische Vorrichtungen (8) in Form übereinanderlegbarer, dehnbarer Elemente unterschiedlicher Abmessungen umfaßt, dessen größtes am weitesten von der Oberfläche des zu behandelnden Gewebes entfernt angeordnet ist und das von Größe und Position her folgende gegen das vorangehende zwischen diesem und dem zu behandelnden Gewebe gesetzt wird und so fort bis zum kleinsten.

5. Vorrichtung zum Strecken von lebendem Gewebe nach einem der Ansprüche 2 bis 4, dadurch gekennzeichnet, daß die dehnbaren Elemente jeweils unterschiedlich dick und breit sind, entsprechend dessen jeweiligen Oberflächenzonen.

6. Vorrichtung zum Strecken von lebendem Gewebe nach Anspruch 1, dadurch gekennzeichnet, daß die elastische Vorrichtung (8) aus einem Gehäuse (11) aus zwei zylindrischen Hohlteilen besteht, die mindestens an einem Ende offen sind und ineinander gleiten, in dem eine beliebige komprimierbare Federvorrichtung (10) angeordnet ist, welche die Teile bis zu einer Maximalspreizposition zu spreizen sucht und die durch ein beliebiges Anschlagsystem begrenzt ist.

7. Vorrichtung zum Strecken von lebendem Gewebe nach Anspruch 1, dadurch gekennzeichnet, daß die elastische Vorrichtung (8) aus einem Gehäuse (11) besteht, das von zwei halbverschlossenen und ineinandergleitenden Teilen gebildet wird, in dem ein beliebiges dehnbares Federsystem (10) angeordnet ist, das die beiden Teile einander anzunähern sucht.

8. Vorrichtung zum Strecken von lebendem Gewebe nach einem der Ansprüche 6 und 7, dadurch gekennzeichnet, daß sie aus mehreren Gehäusen (11) besteht, die mittels Achsen (12) aneinander angelenkt sind, wodurch diese Gehäuse (11) zusammen die natürliche Form des zu behandelnden lebenden Gewebes annehmen können.
